# FASCICULE DE BREVET EUROPEEN

(11) **EP 0 847 986 B1**
(45) Date de publication et mention de la délivrance du brevet: **13.06.2001**
(21) Numéro de dépôt: 97402613.0
(22) Date de dépôt: 03.11.1997
(51) Int. Cl.: C07C 219/06, A61K 7/06

(54) **Dérivés ammoniums quaternaires hydroxypropylés à fonction ester, compositions cosmétiques et dermatologiques les contenant**
Hydroxypropylierte quartäre Ammoniumderivate mit Estergruppe und kosmetische und dermatologische Zusammensetzungen, die sie enthalten.
Esterified hydroxypropylated quaternary ammonium compounds, cosmetic and dermatologic compostions containing them

(30) Priorité: 11.12.1996 FR 9615224
(43) Date de publication de la demande: 17.06.1998
(73) Titulaire: L'OREAL, 75008 Paris (FR)
(72) Inventeur: Philippe, Michel, 91320 Wissous (FR); Campos, Alain, 77290 Mitry-Mory (FR); Semeria, Didier, 77181 Courtry (FR)
(74) Mandataire: Dodin, Catherine

(56) Documents cités:
- FR-A- 1 313 143
- US-A- 3 342 840
- US-A- 4 173 539
- US-A- 4 840 738

## Description

L'invention concerne de nouveaux dérivés d'ammoniums quaternaires hydroxypropylés à fonction ester d'acide gras ainsi que leurs utilisations dans les produits cosmétiques ou dermatologiques notamment dans les produits pour le soin et le traitement des cheveux.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

En effet, sous l'action de ces agressions (agents atmosphériques, traitements mécaniques ou chimiques), les cheveux perdent une partie de leurs constituants, tels que notamment des protéines.

Certains dérivés d'ammoniums quaternaires sont connus depuis de nombreuses années dans les produits capillaires comme agents de conditionnement des cheveux pour faciliter le démêlage et le peignage des cheveux et pour apporter de la douceur. On connaît notamment le bromure de cétyl triméthylammonium et le bromure de béhényltriméthyl ammonium.

On connaît également certains composés ammoniums quaternaires hydroxypropylés à fonction ester d'acide gras comme agents de conditionnement des cheveux conférant un effet brillant, un toucher doux et facilitant le peignage et le démêlage des cheveux. Ils sont décrits dans le brevet FR 1 313 143.

La demanderesse a découvert de manière surprenante une nouvelle famille de dérivés ammoniums quaternaires hydroxypropylés à fonction ester d'acide gras- que l'on définira plus en détail par la suite- permettant, après application sur les cheveux d'obtenir un état de surface du cheveu substantiellement plus lisse ainsi que d'améliorer sensiblement le peignage et le démêlage des cheveux par rapport aux dérivés quaternaires couramment utilisés dans les formulations capillaires.

Les dérivés quaternaires hydroxypropylés à fonction ester de l'invention permettent également d'obtenir une bonne protection de la peau et/ou des fibres capillaires vis à vis des agressions des divers agents atmosphériques et traitements mécaniques et chimiques appliqués généralement sur la peau et/ou les fibres capillaires.

Les dérivés quaternaires hydroxypropylés à fonction ester conformes à l'invention sont caractérisés par le fait qu'ils répondent à la formule générale suivante : 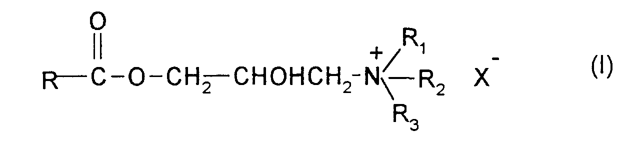 dans laquelle
R désigne une chaîne alkyle ramifiée, saturée ou insaturée en C₇-C₃₅;
R₁, R₂ et R₃, identiques ou différents, désignent une chaîne alkyle, linéaire ou ramifiée, saturée ou insaturée en C₁-C₁₈ ;
X⁻ désigne un halogénure ou un anion choisi dans le groupe constitué par : 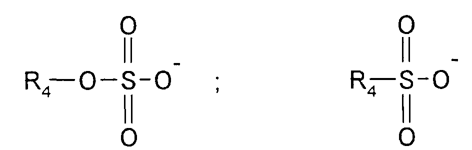 et 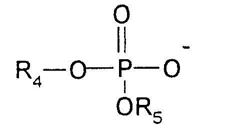
dans lesquels R₄ et R₅, identiques ou différents, désignent une chaîne alkyle, ramifiée ou linéaire, saturée ou insaturée en C₁-C₈.

Les composés préférentiels de formule (I) sont ceux répondant à la formule générale suivante : 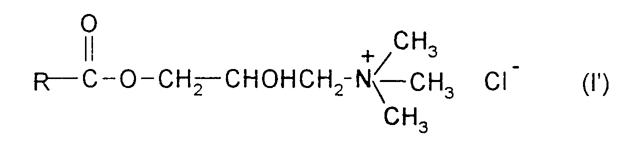 dans laquelle R désigne une chaîne alkyle ramifiée, saturée ou insaturée en C₁₁-C₂₃.

Parmi ces composés, les plus particulièrement préférés sont choisis dans le groupe constitué par le chlorure de [3-(2-octyl-dodécanoyloxy)-2-hydroxypropyl]triméthyl ammonium ; le chlorure de [3-(2-décyl-tetradécanoyloxy)-2-hydroxypropyl]triméthyl ammonium ; le chlorure de [3-(2-butyl-octanoyloxy)-2-hydroxypropyl]-triméthyl ammonium ; le chlorure de [3-(2-hexyl-décanoyloxy)-2-hydroxypropyl]-triméthyl ammonium.

Les composés de formule (I) de l'invention peuvent être préparés selon un procédé en deux étapes décrit dans le brevet FR 1 313 143 et consistant :
(1°) à faire réagir un sel alcalin d'acide carboxylique aliphatique gras avec une épihalohydrine, en particulier l'épichlorhydrine, et une amine secondaire dans un solvant alcoolique (isopropanol ou tertio butanol) et
(2°) à séparer l'halogénure de métal alcalin formé et à effectuer une quaternisation de l'aminoester formé dans la première étape par un agent alkylant tel que le sulfate de diméthyle.

Les composés de formule (I) de l'invention peuvent être également préparés selon un procédé en deux étapes décrit dans le brevet US 3 872 138 et consistant à faire réagir un sel d'amine tertiaire avec un acide carboxylique dans des quantités stoechiométriques pour neutraliser l'acide par l'amine puis à faire réagir le sel résultant avec de l'épichlorhydrine dans un solvant du type benzène à une température de 50 à 150°C pendant une durée en général de 15 heures.

De façon préférentielle, les composés de formule (I) de l'invention peuvent être préparés selon un procédé en une seule étape dans des conditions de mise en oeuvre plus faciles, plus rapides et plus sécurisantes que celles des deux procédés définis précédemment sans utiliser d'épihalohydrine, d'agent alkylant généralement toxique ni de solvant à haute toxicité.

Ce procédé consiste à faire réagir, dans un solvant alcoolique à reflux, un sel d'acide carboxylique aliphatique gras RCOOH ou un acide carboxylique aliphatique en catalyse basique avec un composé de formule (II) suivante : 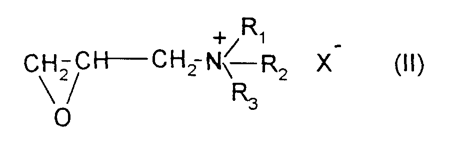 où R, R₁, R₂, R₃ et X⁻ ont les mêmes significations indiquées dans la formule (I) ci-dessus.

Le sel d'acide carboxylique est de préférence un sel de métal alcalin ou alcalino-terreux d'acide carboxylique. Les sels de métal alcalin tels que de sodium sont particulièrement préférés. La catalyse basique est de préférence réalisée avec la soude, la potasse ou la triéthylamine. Le solvant alcoolique utilisé est de préférence choisi parmi les alcools inférieurs en C₁-C₄ et plus particulièrement le 2-butanol. La température de réaction est en général comprise entre 45 et 150°C et la durée de réaction varie de préférence de 4 à 8 heures. Selon ce procédé, le produit final obtenu en fin de réaction peut être ensuite purifié par simple lavage et/ou simple extraction à base d'un solvant choisi par exemple parmi l'heptane, un alcool inférieur en C₁-C₄ tel que le méthanol ou leurs mélanges.

Un autre objet de l'invention consiste en des compositions cosmétiques ou dermatologiques caractérisées par le fait qu'elles contiennent dans un milieu cosmétiquement acceptable au moins un composé de formule (I).

On entend par milieu cosmétiquement acceptable, tout milieu compatible avec toutes les matières kératiniques telles que la peau, les ongles, les muqueuses et les cheveux ou toute autre zone cutanée du corps.

Les compositions de l'invention contiennent les composés de formule (I) de préférence dans des concentrations allant de 0,001 et 20% en poids et plus particulièrement de 0,05 à 15% en poids par au poids total de la composition.

La composition de l'invention peut également contenir au moins un additif choisi parmi les épaississants, les silicones volatiles ou non volatiles, solubles ou insolubles, les tensioactifs, les parfums, les agents nacrants, les conservateurs, les filtres solaires, les protéines, les vitamines, les polymères, les solvants couramment utilisés en cosmétologie ou dermatologie, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20% en poids par rapport au poids total de la composition. La quantité précise de chaque additif est déterminée facilement par l'homme de l'art selon sa nature et sa fonction.

L'invention a encore pour objet un procédé de traitement de la peau ou des fibres kératiniques, telles que les cheveux, caractérisé en ce qu'il consiste à appliquer sur la peau ou sur les fibres kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage de la peau, des cheveux ou de toute autre matière kératinique.

Les compositions cosmétiques selon l'invention peuvent se présenter sous forme de gel, de lait, de crème, de lotion ou solution plus ou moins épaissie ou de mousse et être utilisées pour la peau ou les cheveux.

Pour les cheveux, elles sont plus particulièrement des shampooings, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions peuvent être également des lotions de mise en plis, des lotions pour le brushing, des compositions de fixation (laques) et/ou de coiffage. Les lotions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote ou l'air comprimé.

L'invention a également pour objet l'utilisation des composés de formule (I) tels que définis ci-dessus comme agent de conditionnement des cheveux dans la préparation d'une formulation pour le soin et/ou le traitement des cheveux et plus particulièrement comme agent permettant d'améliorer le lissage, le peignage et le démêlage des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

### EXEMPLES DE PREPARATION

### EXEMPLE 1: Préparation du chlorure de [3-(2-octyl-dodécanoyloxy)-2-hydroxypropyl]-triméthyl ammonium

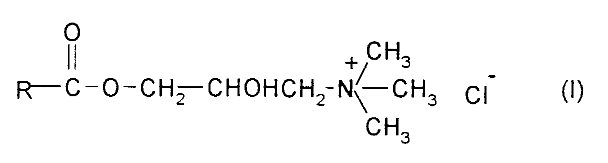

avec

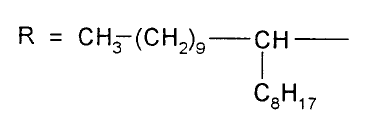

Dans un ballon muni d'une agitation, d'un thermomètre, d'une ampoule de coulée et d'un réfrigérant ascendant, on introduit :
- 100 ml de 2-butanol,
- 0,15 mole d'acide 2-octyl-dodécanoïque (46,5g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le mileu réactionnel est lavé à l'eau puis à l'heptane. La phase supérieure est concentrée à sec puis reprise dans un mélange heptane/méthanol/eau. La phase inférieure est concentrée à sec. On obtient une pâte ambrée.

### Analyses

Indice de chlorure : 1,95 meq/g
Indice d'acide : 0,43 meq/g soit 19% acide (en mole)

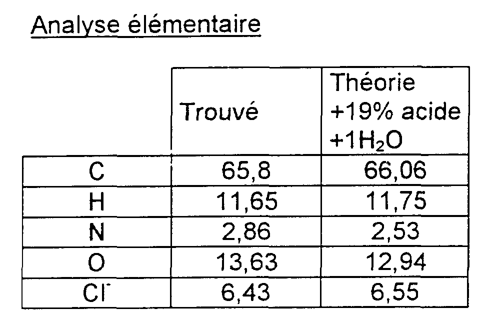

### EXEMPLE 2: Préparation du chlorure de [3-(2-décyl-tetradécanoyloxy)-2-hydroxypropyl]-triméthyl ammonium

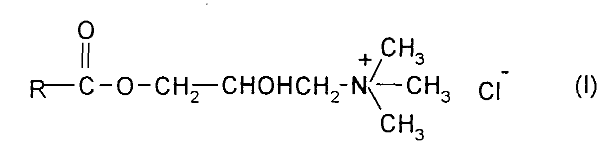

avec

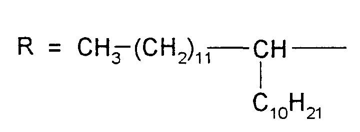

Dans un ballon muni d'une agitation, d'un thermomètre, d'une ampoule de coulée et d'un réfrigérant ascendant, on introduit :
- 100 ml de 2-butanol,
- 0,15 mole d'acide 2-décyl-tetradécanoïque (36,8g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le milieu réactionnel est lavé à l'eau puis à l'heptane. La phase supérieure est concentrée à sec puis reprise dans un mélange heptane/méthanol/eau. La phase inférieure est concentrée à sec. On obtient une pâte jaune clair.

### Analyses

Indice de chlorure : 1,71 meq/g
Indice d'acide : 0,174 meq/g soit 10% acide (en mole)

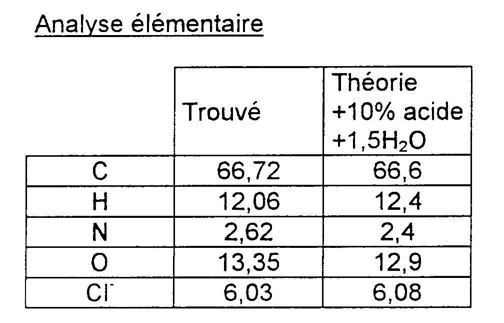

### EXEMPLE 3 : Préparation du chlorure de [3-(2-butyl-octanoyloxy)-2-hydroxypropyl]-triméthyl ammonium

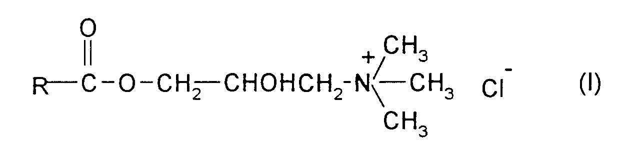

avec

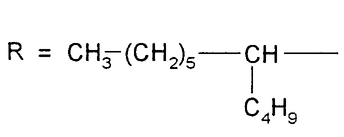

Dans un ballon muni d'une agitation, d'un thermomètre, d'une ampoule de coulée et d'un réfrigérant ascendant, on introduit :
- 100 ml de 2-butanol,
- 0,15 mole d'acide 2-butyl-octanoïque (30g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le milieu réactionnel est lavé à l'eau puis à l'heptane. La phase supérieure est concentrée à sec puis reprise dans un mélange heptane/méthanol/eau. La phase inférieure est concentrée à sec. On obtient une pâte ambrée.

### Analyses

Indice de chlorure : 2,60 meq/g
Indice d'acide : 0,11 meq/g soit 4,2% acide (en mole)

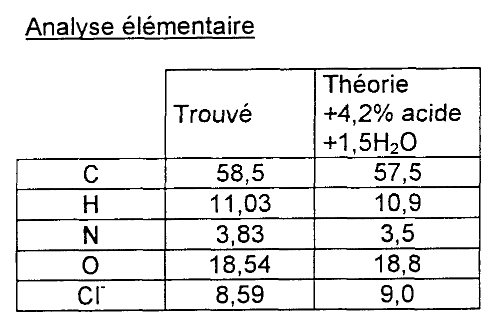

### EXEMPLE 4 : Préparation du chlorure de [3-(2-hexyl-décanoyloxy)-2-hydroxypropyl]-triméthyl ammonium

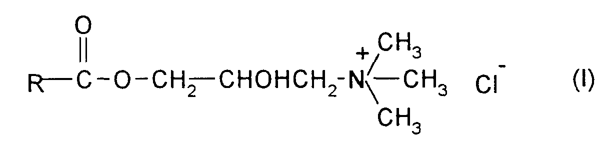

avec

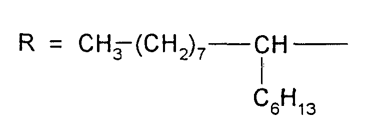

Dans un ballon muni d'une agitation, d'un thermomètre, d'une ampoule de coulée et d'un réfrigérant ascendant, on introduit :
- 100 ml de 2-butanol,
- 0,15 mole d'acide 2-hexyl-décanoïque (38,4g),
- 0,075 mole d'hydrogénocarbonate de sodium (0,63g).

On chauffe au reflux du 2-butanol (98-99°C).

Dès que l'hydrogénocarbonate s'est solubilisé dans le milieu, on introduit goutte à goutte 0,15 mole (22,7g) de chlorure de glycidyle-triméthyl ammonium dissous dans 50 ml de 2-butanol. Le reflux est maintenu pendant 6 heures après la fin de l'introduction.

Le milieu réactionnel est lavé à l'eau puis à l'heptane. La phase supérieure est concentrée à sec puis reprise dans un mélange heptane/méthanol/eau. La phase inférieure est concentrée à sec. On obtient une pâte ambrée.

### Analyses

Indice de chlorure : 1,98 meq/g
Indice d'acide : 0,066 meq/g soit 3,3% acide (en mole)

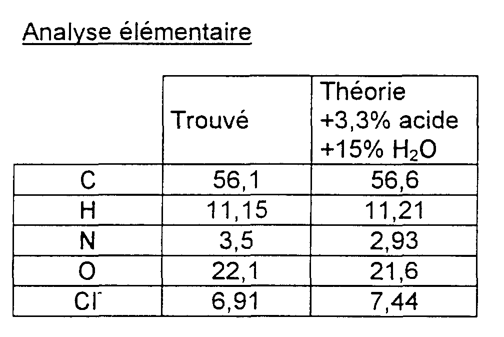

### EXEMPLES D'APPLICATION

### EXEMPLE A : Shampooing

- Lauryl éther sulfate de sodium 12 g
- Composé de l'exemple 4 0,5 g M.A.
- Colorants, parfums, conservateurs qs
- Eau pH ajusté à 7 qsp 100 g

Ce shampooing possède un bon pouvoir moussant, améliore le démêlage et la douceur des cheveux.

## Revendications

1. Dérivés d'ammoniums quaternaires hydroxypropylés à fonction ester d'acide gras de formule (I) suivante : 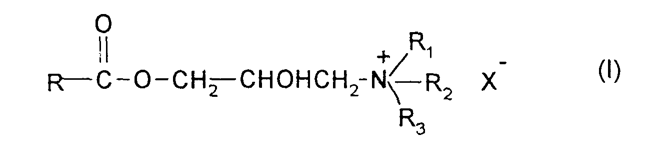 dans laquelle :
R désigne une chaîne alkyle ramifiée, saturée ou insaturée en C₇-C₃₅ ;
R₁, R₂ et R₃, identiques ou différents, désignent une chaîne alkyle, ramifiée ou linéaire, saturée ou insaturée en C₁-C₁₈ ;
X⁻ désigne un halogénure(préférentiellement le chlore) ou un anion choisi dans le groupe constitué par : 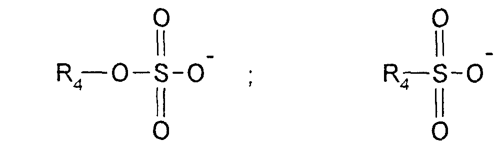 et 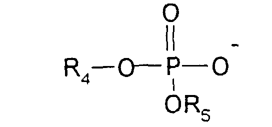
dans lesquels R₄ et R₅, identiques ou différents, désignent une chaîne alkyle, ramifiée ou linéaire, saturée ou insaturée en C₁-C₈

2. Dérivés d'ammoniums quaternaires hydroxypropylés à fonction ester d'acide gras répondant à la formule générale suivante : 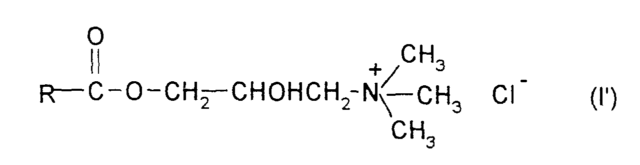 dans laquelle R désigne une chaîne alkyle ramifiée, saturée ou insaturée en C₁₁-C₂₃.

3. Dérivés selon la revendication 1 ou 2 choisis dans le groupe constitué par le chlorure de [3-(2-octyl-dodécanoyloxy)-2-hydroxypropyl]-triméthyl ammonium le chlorure de [3-(2-décyl-tetradécanoyloxy)-2-hydroxypropyl]-triméthyl ammonium ; le chlorure de [3-(2-butyl-octanoyloxy)-2-hydroxypropyl]-triméthyl ammonium le chlorure de [3-(2-hexyl-décanoyloxy)-2-hydroxypropyl]-triméthyl ammonium.

4. Composition cosmétique ou dermatologique, caractérisée par le fait qu'elle contient dans un mileu cosmétiquement acceptable au moins un composé de formule (I) tels que définis selon l'une quelconque des revendications 1 à 3.

5. Composition selon la revendication 4, contenant au moins un composé de formule (I) dans des concentrations allant de 0,001 et 20% en poids et plus particulièrement allant de 0,05 à 15% en poids par au poids total de la composition.

6. Composition selon l'une quelconque des revendications 4 et 5, caractérisée par le fait qu'elle se présente sous forme de gel, de lait, de crème, de lotion ou solution plus ou moins épaissie ou de mousse.

7. Composition selon l'une quelconque des revendications 4 à 6, caractérisée par le fait qu'il s'agit d'un produit capillaire.

8. Composition selon la revendication 7, caractérisée par le fait qu'il s'agit d'un produit capillaire choisi parmi les shampooings ; les compositions à rincer ou non, à appliquer avant ou après un shampooing, une coloration, une décoloration, une permanente ou un défrisage ; les compositions de coloration ; les compositions de décoloration ; les compositions de permanente ou de défrisage des cheveux ; les lotions de mise en plis ; les lotions pour le brushing ; les compositions de fixation et/ou de coiffage.

9. Composition selon l'une quelconque des revendications 4 à 8, caractérisée par le fait qu'elle est conditionnée sous forme d'aérosol en vue d'obtenir une laque ou une mousse aérosol et comprend au moins un agent propulseur.

10. Composition selon la revendication 9, caractérisée par le fait que l'agent propulseur est choisi parmi les hydrocarbures volatils, les hydrocarbures chlorés et/ou fluorés et leurs mélanges ; le protoxyde d'azote ; le diméthyléther ; l'azote ou l'air comprimé.

11. Procédé de traitement de la peau ou des fibres kératiniques, telles que les cheveux, caractérisé en ce qu'il consiste à appliquer sur la peau ou sur les fibres kératiniques une composition cosmétique telle que définie dans l'une quelconque des revendications 4 à 10, puis à effectuer éventuellement un rinçage à l'eau.

12. Utilisation des composés de formule (I) tels que définis dans l'une quelconque des revendications 1 à 3 comme agent de conditionnement des cheveux dans et pour la préparation d'une composition pour le soin et/ou le traitement des cheveux.

13. Utilisation des composés de formule (I) tels que définis dans l'une quelconque des revendications 1 à 3 comme agent permettant d'améliorer le lissage, le peignage et le démêlage des cheveux dans et pour la préparation d'une composition pour le soin et/ou le traitement des cheveux.

## Patentansprüche

1. Hydroxypropylierte quartäre Ammoniumderivate mit Fettsäureestergruppe der folgenden Formel (I): 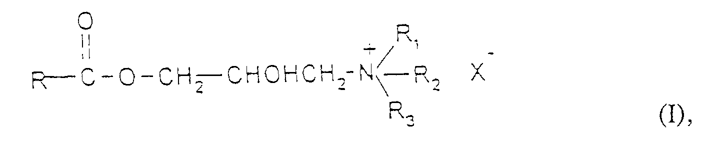 worin bedeuten:
- R eine verzweigte, gesättigte oder ungesättigte C₇₋₃₅-Alkylgruppe,
- R₁, R₂ und R₃, die identisch oder voneinander verschieden sind, eine geradkettige oder verzweigte, gesättigte oder ungesättigte C₁₋₁₈-Alkylgruppe,
- X⁻ ein Halogenid (vorzugsweise Chlor) oder ein Anion, das unter den folgenden Verbindungen ausgewählt ist: 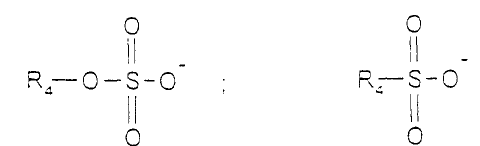 und 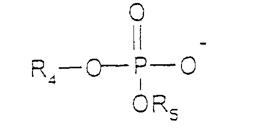
worin die Gruppen R₄ und R₅, die identisch oder voneinander verschieden sind, eine geradkettige oder verzweigte, gesättigte oder ungesättigte C₁₋₈-Alkylgruppe bedeuten.

2. Hydroxypropylierte quartäre Ammoniumderivate mit Fettsäureestergruppe, die der folgenden allgemeinen Formel entsprechen: 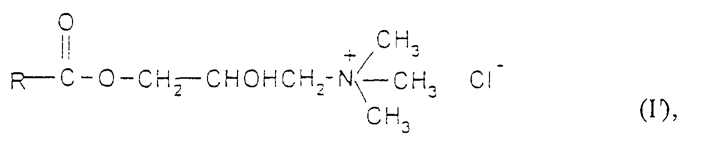 worin R eine verzweigte, gesättigte oder ungesättigte C₁₁₋₂₃-Alkylgruppe bedeutet.

3. Derivate nach Anspruch 1 oder 2, die unter [3-(2-Octyl-dodecanoyloxy)-2-hydroxypropyl]-trimethylammoniumchlorid, [3-(2-Decyl-tetradecanoyloxy)-2-hydroxypropyl]-trimethylammoniumchlorid, [3-(2-Butyl-octanoyloxy)-2-hydroxypropyl]-trimethylammoniumchlorid und [3-(2-Hexyl-decanoyloxy)-2-hydroxypropyl]-trimethylammoniumchlorid ausgewählt sind.

4. Kosmetische oder dermatologische Zusammensetzung, dadurch gekennzeichnet, dass sie in einem kosmetisch akzeptablen Medium mindestens eine in einem der Ansprüche 1 bis 3 definierte Verbindung der Formel (I) enthält.

5. Zusammensetzung nach Anspruch 4, die mindestens eine Verbindung der Formel (I) in Konzentrationen von 0,001 bis 20 Gew.-% und insbesondere von 0,05 bis 15 Gew.-%, bezogen auf das Gesamtgewicht der Zusammensetzung, enthält.

6. Zusammensetzung nach einem der Ansprüche 4 und 5, dadurch gekennzeichnet, dass sie als Gel, Milch, Creme, Lotion oder mehr oder weniger dickflüssige Lösung oder als Schaum vorliegt.

7. Zusammensetzung nach einem der Ansprüche 4 bis 6, dadurch gekennzeichnet, dass es sich um ein Produkt für die Haare handelt.

8. Zusammensetzung nach Anspruch 7, dadurch gekennzeichnet, dass es sich um ein Produkt für die Haare handelt, das unter Haarwaschmitteln, Zusammensetzungen, die ausgespült oder nicht ausgespült werden, die vor oder nach einer Haarwäsche, Färbung, Entfärbung, permanenten Verformung oder Entkräuselung aufgebracht werden, Zusammensetzungen zum Färben, Zusammensetzungen zum Entfärben, Zusammensetzungen zur permanenten Verformung oder zur Entkräuselung der Haare, Lotionen für Wasserwellen, Lotionen für Fönwellen, Zusammensetzungen zum Fixieren und/oder zum Frisieren ausgewählt ist.

9. Zusammensetzung nach einem der Ansprüche 4 bis 8, dadurch gekennzeichnet, dass sie in Form eines Aerosols konfektioniert ist, um einen Lack oder einen Aerosolschaum zu erhalten, und mindestens ein Treibmittel enthält.

10. Zusammensetzung nach Anspruch 9, dadurch gekennzeichnet, dass das Treibmittel unter den flüchtigen Kohlenwasserstoffen, chlorierten und/oder fluorierten Kohlenwasserstoffen und deren Gemischen, Distickstoffoxid, Dimethylether, Stickstoff oder Druckluft ausgewählt ist.

11. Verfahren zur Behandlung der Haut oder von Keratinfasern, wie dem Haar, dadurch gekennzeichnet, dass es darin besteht, eine in einem der Ansprüche 4 bis 10 definierte kosmetische Zusammensetzung auf die Haut oder die Keratinfasern aufzubringen und dann gegebenenfalls mit Wasser zu spülen.

12. Verwendung der in einem der Ansprüche 1 bis 3 definierten Verbindungen der Formel (I) als Mittel zur Konditionierung der Haare in einer Zusammensetzung zur Pflege und/oder zur Behandlung der Haare und zur Herstellung dieser Zusammensetzung.

13. Verwendung der in einem der Ansprüche 1 bis 3 definierten Verbindungen der Formel (I) als Mittel, mit dem das Glätten, Kämmen und Ausbürsten des Haares erleichtert werden kann, in einer Zusammensetzung zur Pflege und/oder zur Behandlung der Haare und zur Herstellung dieser Zusammensetzung.

## Claims

1. Hydroxypropyl quaternary ammonium derivatives containing a fatty acid ester function, of formula (I) below: 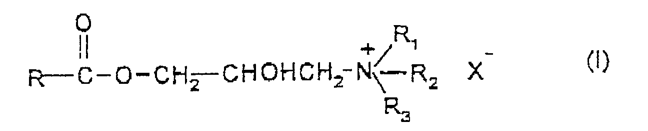 in which
R denotes a saturated or unsaturated, branched C₇-C₃₅ alkyl chain;
R₁, R₂ and R₃, which may be identical or different, denote a saturated or unsaturated, linear or branched C₁-C₁₈ alkyl chain;
X⁻ denotes a halide (preferably chloride) or an anion chosen from the group consisting of: 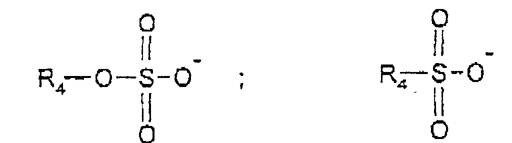 and 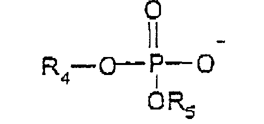
in which R₄ and R₅, which may be identical or different, denote a saturated or unsaturated, linear or branched C₁-C₈ alkyl chain.

2. Hydroxypropyl quaternary ammonium derivatives containing a fatty acid ester function, corresponding to the following general formula: 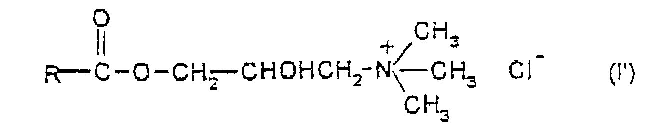 in which R denotes a saturated or unsaturated, branched C₁₁-C₂₃ alkyl chain.

3. Derivatives according to Claim 1 or 2, chosen from the group consisting of [3-(2-octyldodecanoyloxy)-2-hydroxypropyl]trimethylammonium chloride; [3-(2-decyltetradecanoyloxy)-2-hydroxypropyl]trimethylammonium chloride; [3-(2-butyl-octanoyloxy)-2-hydroxypropyl]trimethylammonium chloride; [3-(2-haxyldecanoyloxy)-2-hydroxypropyl]trimethylammonium chloride.

4. Cosmetic or dermatological composition, characterized in that it contains at least one compound of formula (I) as defined according to any one of Claims 1 to 3 in a cosmetically acceptable medium.

5. Composition according to Claim 4, containing at least one compound of formula (I) in concentrations ranging from 0.001 to 20% by weight, and more particularly ranging' from 0.05 to 15% by weight, relative to the total weight of the composition.

6. Composition according to either of Claims 4 and 5, characterized in that it is in the form of a gel, a milk, a cream, a lotion or solution which is relatively thickened, or a foam.

7. Composition according to any one of Claims 4 to 6, characterized in that it is a haircare product.

8. Composition according to Claim 7, characterized in that it is a haircare product chosen from shampoos; rinse-out or leave-in compositions to be applied before or after a shampooing, dyeing, bleaching, permanent-waving or hair-straightening operation; dyeing compositions; bleaching compositions; permanent-waving or straightening compositions for the hair; hairsetting lotions; blow-drying lotions; fixing and/or styling compositions.

9. Composition according to any one of Claims 4 to 8, characterized in that it is packaged in the form of an aerosol in order to obtain an aerosol lacquer or foam and comprises at least one propellant.

10. Composition according to Claim 9, characterized in that the propellant is chosen from volatile hydrocarbons, chloro and/or fluoro hydrocarbons and mixtures thereof; nitrous oxide; dimethyl ether; nitrogen or compressed air.

11. Process for treating the skin or keratin fibres, such as the hair, characterized in that it consists in applying a cosmetic composition as defined in any one of Claims 4 to 10 to the skin or to the keratin fibres and then optionally in rinsing with water.

12. Use of the compounds of formula (I) as defined in any one of Claims 1 to 3 as a hair conditioner in, and for the preparation of, a haircare and/or hair treatment composition.

13. Use of the compounds of formula (I) as defined in any one of Claims 1 to 3 as an agent for improving the smoothness, combing and disentangling of the hair in, and for the preparation of, a haircare and/or hair treatment composition.
